# EUROPÄISCHE PATENTANMELDUNG

(11) **EP 0 789 075 A1**
(43) Veröffentlichungstag der Anmeldung: **13.08.1997**
(21) Anmeldenummer: 97100593.9
(22) Anmeldetag: 16.01.1997
(51) Int. Cl.: C12N 9/28, C12N 9/34

(54) **Enzymgemische und Verfahren zur Entschlichtung von mit Stärke geschlichteten Textilien**

(30) Priorität: 29.01.1996 DE 19603054
(71) Anmelder: BAYER AG, 51368 Leverkusen (DE); Genencor International GmbH, 31564 Nienburg (DE)
(72) Erfinder: Hahn, Wilfried, 31582 Nienburg-Weser (DE); Seitz, Axel, 30966 Hemmingen (DE); Riegels, Martin, Dr., 42799 Leichlingen (DE); Koch, Rainhard, Dr., 51065 Köln (DE); Pirkotsch, Michael, 51381 Leverkusen (DE)
(74) Vertreter: Zobel, Manfred, Dr.

(57) **Zusammenfassung**

Gemische verschiedener stärkeabbauender Enzyme (Amylasen), die aus mindestens einer Hochtemperaturamylase (HTA) und mindestens einer Niedertemperaturamylase (NTA) im Aktivitätsverhältnis von HTA zu NTA von 10 % : 90 % bis 90 % : 10 % bestehen, entwickeln im Temperaturbereich von 30 bis 90°C mindestens 60 % ihrer maximalen Aktivität. Solche Gemische können mit Wasser verdünnt und mit üblichen Zusatzmitteln versetzt werden. Diese Gemische eignen sich zum Entschlichten von mit Stärke geschlichteten Textilien durch Behandlung der Textilien mit den genannten Gemischen und anschließendem Spülen.

## Beschreibung

Die Erfindung betrifft Gemische verschiedener stärkeabbauender Enzyme (Amylasen), die aus mindestens einer Hochtemperaturamylase und mindestens einer Niedertemperaturamylase bestehen. Die Erfindung betrifft ferner ein Verfahren zum Entschlichten von mit Stärke geschlichteten Textilien, in welchem diese Textilien mit den genannten Enzymgemischen behandelt werden.

Textilien werden vor der Gewebeherstellung mit Schlichten beaufschlagt. Die Schlichten verbessern oder ermöglichen erst die maschinentechnische Verarbeitung der Garne in der Weberei bei hohen Maschinengeschwindigkeiten. Dabei ist die Kette des Gewebes im Verlauf des Webens deutlich höheren mechanischen Beanspruchungen ausgesetzt als der Schuß. Zur Vermeidung von Gernbrüchen muß das Garn daher vor dem Webprozeß geschlichtet werden. Als Schlichten können verschiedene Materialien eingesetzt werden, wie beispielsweise Gelatine, Leinsamenöl, Johannisbrotgummi sowie vermehrt in den letzten Jahren auch synthetische Materialien, wie Polyvinylalkohole, Polyacrylate und wasserlösliche Cellulosederivate, wie Carboxymethylcellulose. Obwohl vor allen Dingen die zuletzt genannten synthetisch hergestellten Schlichtemittel technologische Vorteile aufweisen, ist aus ökologischen Gründen Stärke auch heute noch eine der wichtigsten Schlichten. In Europa wird vor allen Dingen Kartoffelstärke eingesetzt, während in Übersee große Mengen an Mais- und Reisstärke Verwendung finden.

Aufgrund der unterschiedlichen mechanischen Beanspruchungen besitzen die geschlichtete Kette und der geschlichtete Schuß von Geweben unterschiedliche Eigenschaften, die sich beispielsweise im Bleichprozeß, während des Färbens und in der weiteren Ausrüstung negativ bemerkbar machen. Aus diesem Grund ist die Entschlichtung der Gewebe vor jeder weiteren Verarbeitung unbedingt erforderlich. Während gut wasserlösliche Schlichten bereits durch eine heiße Wäsche entfernt werden können, widersteht Stärke diesem einfachen Prozeß. Die Stärke muß in eine wasserlösliche Form übergeführt werden, um ausgewaschen werden zu können. In früheren Anwendungsjahren der Stärke erzielte man durch Behandlung mit verdünnter Schwefelsäure den gewünschten Effekt der Entschlichtung. Diese Behandlung schädigte jedoch sehr stark das Gewebe. Aus diesem Grund setzte sich die faserschonende enzymatische Entschlichtung am Markt schnell durch.

Für die enzymatische Entschlichtung sind derzeit unterschiedliche stärkeabbauende Enzyme (Amylasen) verfügbar, die entweder bei Temperaturen von 30 bis 70°C (Niedertemperaturamylasen) oder bei Temperaturen von 70 bis 110°C (Hochtemperaturamylasen) aktiv sind. Sie können aus Bakterien, Pilzen, Pflanzen oder Tieren gewonnen werden. Bei den Niedertemperaturamylasen handelt es sich oftmals um stärkeabbauende Enzyme, welche aus Bacillus subtilis oder Bacillus amyloliquefaciens stammen. Entsprechende Enzyme aus Aspergillus oryzae können ebenfalls eingesetzt werden. Die Hochtemperaturamylasen entstammen oftmals dem Bakterium Bacillus licheniformis. Je nachdem, bei welcher Temperatur die Entschlichtung durchgeführt wird, müssen also entweder Hoch- oder Niedertemperaturamylasen für den Prozeß ausgewählt werden. Produkte, welche stärkeabbauende Enzyme enthalten und universell bei allen üblichen Temperaturbereichen Aktivität besitzen, gibt es bislang nicht.

In DE-A 29 09 396 wird ein Entschlichtungsmittel und ein Verfahren zu seiner Herstellung beschrieben. Das Hilfsmittel besteht aus einer innigen Mischung eines stärkeabbauenden Enzyms mit einem Tensid in Wasser. Mit der beschriebenen Mischung kann auf den sonst üblichen Zusatz eines Tensids während der Entschlichtung verzichtet werden. Das Entschlichtungsmittel kann jedoch nicht über den gesamten Temperaturbereich erfolgreich eingesetzt werden, sondern erfordert eine Anwendungstemperatur von 90°C bis Kochtemperatur. JP 06/235 163 (1987; zitiert nach C.A.121 (1994), 282294q) und JP 02/80 673 (1990; zitiert nach C.A. 113 (1990), 61270 m) beschreiben die enzymatische Entschlichtung mit Amylasen bei 100 bis 115°C bzw. 50°C. Wegen der sehr guten Hitzestabilität eignet sich die in WO 94/19454 beschriebene Amylase zur Entschlichtung bei hohen Temperaturen. DE-A 28 36 516 beschreibt ein Verfahren zum Kaltentschlichten von Textilien mit α-Amylasen. In WO 91/19794 wird eine verbesserte enzymatische Entschlichtung mit α-Amylasen unter Zusatz von nicht-ionischen Tensiden beschrieben. Eine gleichzeitige Wasserstoffperoxidbleiche und enzymatische Entschlichtung beschreiben die Anmeldungen EP-A 55 664, EP-A 119 920 und US 4.643.736 (Natriumhypochloritbleiche) sowie DE-A 27 35 816 (H₂O₂). US 4.371.372 beschreibt einen kombinierten Färbe- und enzymatischen Entschlichtungsprozeß bei Temperaturen unterhalb von 30°C.

Nun ist es tägliche Praxis in Textilverarbeitungsunternehmen, daß leichte Textilware, die kalt entschlichtet werden kann, mit schwerer Ware, bei der eine Kaltentschlichtung unzureichend ist, abwechselt. Weiterhin bringt die Auftragssituation in unregelmäßiger Folge sowohl kleine Auftragspartien, bei denen man wiederum die Kaltentschlichtung vorzieht, als auch große Auftragspartien, bei denen eine Kontinue-Behandlung unter Hochtemperaturbedingungen ökonomischer ist. Es besteht daher ein Bedarf nach enzymatischen Entschlichtungsmitteln, die universell sowohl in der Hoch- als auch in der Niedertemperaturtechnik der Entschlichtung anwendbar und in der Lagerhaltung ökonomischer sind.

Es wurde nun gefunden, daß dieser Forderung durch den Einsatz der weiter unten beschriebenen erfindungsgemäßen Enzymgemische entsprochen werden kann. Es wurde dabei zusätzlich überraschend gefunden, daß die für das Arbeiten in einem der beiden Temperaturbereiche vermeintlich nicht geeigneten Enzyme des Gemisches keinewegs stören, was immerhin durch ihre Trägheit im vermeintlich "falschen" Temperaturbereich und durch evtl. Zersetzungs- oder Abbauprodukte in diesem "falschen" Temperaturbereich zu erwarten gewesen wäre. Es wurde vielmehr gefunden, daß die Gegenwart der für zwei verschiedene Temperaturbereiche geeigneten Enzyme in der Praxis einen unerwarteten Synergismus ausübt, der sich darin äußert, daß das erfindungsgemäße Gemisch in geringerer Menge eingesetzt zu werden braucht als ein vergleichbares Spezialenzym, um den gewünschten Effekt zu erzielen. Dies stellt einen wirtschaftlichen Vorteil dar, der über die blose Vorratshaltung nur eines statt zweier Entschlichtungsmittel weit hinausgeht.

Die Erfindung betrifft Gemische verschiedener stärkeabbauender Enzyme (Amylasen), die aus mindestens einer Hochtemperaturamylase (HTA) und mindestens einer Niedertemperaturamylase (NTA) im Aktivitätsverhältnis von HTA zu NTA von 10 % : 90 % bis 90 % : 10 % bestehen. Sie besitzt im Temperaturbereich von 30 bis 90°C mehr als 60 % ihrer max. Aktivität und kann weiterhin mit Wasser verdünnt und mit üblichen Zusatzmitteln versetzt werden.

Das Verhältnis, in welchem HTA und NTA gemischt werden, richtet sich nach deren Aktivität im Temperatur- und pH-Optimum. Dies wird nach H.U. Bergmeyer bestimmt (H.U. Bergmeyer, Methods for Enzymatic Analysis, 3rd ed., Vol. 2, S. 151-152, Verlag Chemie GmbH, Weinheim). Als NTA werden Amylasen mit einer maximalen Aktivität im Temperaturbereich von 30 bis 70°C bezeichnet; als HTA werden Amylasen mit einer maximalen Aktivität im Temperaturbereich von 70 bis 110°C bezeichnedt. Die maximale Aktivität der einzelnen marktgängigen NTA bzw. HTA liegen jeweils in einem sehr viel engeren und für die einzelnen Amylasen spezifischen Temperaturbereich.

Die erfindungsgemäßen Gemische enthalten Aktivitätsanteile von 10 % HTA und 90 % NTA bis zu 90 % HTA und 10 % NTA. Die Aktivitätsverhältnisse betragen bevorzugt HTA : NTA = 20 % : 80 % bis 80 % : 20 %, besonders bevorzugt 30 % : 70 % bis 70 % : 30 %, ganz besonders bevorzugt 40 % : 60 % bis 60 % : 40 %. Solche Gemische entwickeln im Bereich von 30 bis 90°C 60 % ihre maximale Aktivität und füllen demnach gegenseitig die Aktivitätslücke zwischen den Aktivitätsmaxima der HTA und der NTA auf. Es ist möglich, geringere Mengen an HTA/NTA-Gemisch einzusetzen, als mit einer konventionellen Amylase nötig wäre (s. Beispiele.). Es ist weiterhin möglich, weniger aktive und damit preiswertere Amylasen für die erfindungsgemäßen Gemische einzusetzen. Noch weiter ist es möglich, nur ein erfindungsgemäßes Enzymgemisch für einen großen Temperaturbereich vorzuhalten statt vieler Enzyme, die jeweils nur in einem spezifischen und engen Temperaturbereich ausreichend aktiv sind.

Die erfindungsgemäßen Gemische können wie andere Enzyme mit Wasser verdünnt und mit den üblichen Zusatzmitteln versetzt werden.

In bevorzugter Weise haben die erfindungsgemäßen Gemische ferner mindestens 80 % der maximalen Aktivität im Temperaturbereich von 45 bis 75°C.

Die Erfindung betrifft weiterhin ein Verfahren zum Entschlichten von mit Stärke geschlichteten Textilien durch Behandlung der Textilien mit stärkeabbauenden Enzymen (Amylasen) und anschließendem Spülen, das dadurch gekennzeichnet ist, daß man die Behandlung bei 30 bis 98°C mit einem Gemisch der oben beschriebenen Art vornimmt.

Die Entschlichtung kann sowohl diskontinuierlich (z.B. Jigger, KKV) als auch kontinuierlich (z.B. Dämpfer) durchgeführt werden.

Die erfindungsgemäßen Gemische können durch einfaches Mischen der handelsüblichen Enzyme bei Raumtemperatur hergestellt werden. Die Gemische können sowohl mit entmineralisiertem als auch mit normalem Leitungswasser beliebig verdünnt werden. Die Gemische können ferner übliche Stell- und Konservierungsmittel enthalten, beispielsweise Alkohole, Glykole oder Glykolether, wie 1-Methoxy-2-propanol, Isopropanol, Butyldiglykol, Natriumbenzoat, Calciumsalze und Isothiazolone, z.B. 5-Chlor-2-methyl-3(2H)-isothiazolon oder 2-Methyl-3(2H)isothiazolon.

Als erfindungsgemäß zu entschlichtende Textilien kommen beispielsweise solche aus Baumwolle und Baumwoll-Mischgeweben in Frage; Baumwoll-Mischgewebe sind etwa solche von Baumwolle mit Polyester, Polyamid, Polyacrylnitril oder anderen cellulosischen Fasern, wie Regeneratcellulose.

Die Aktivität der erfindungsgemäßen Gemische kann beispielsweise mit löslicher Stärke als Substrat bei verschiedenen Temperaturen im obengenannten Bereich bestimmt werden. Die Daten der folgenden Tabelle 1 wurden mit einem erfindungsgemäßen Entschlichtungsmittel (Gemisch) aus einer NTA aus Bacillus amyloliquefaciens und einer HTA aus Bacillus licheniformis erstellt. Die mit dem Gemisch erhaltenen Daten werden verglichen mit denen einer weiteren HTA (Aquazym 250 L Fa. NOVO Nordisk), die konventionell ist und zum Entschlichten als Einzelenzym bereits eingesetzt wird. Sie ist nicht identisch mit der HTA, die im erfindungsgemäßen Gemisch enthalten ist.

Das genaue Verhältnis, in dem Enzyme aus dem Bereich der HTA und dem der NTA gemischt werden, richtet sich im Einzelfall nach ihrem Temperatur- und pH-Optimum; dies kann durch einfache Vorversuche bestimmt werden. Die Aktivitäten können z.B. nach H.U. Bergmeyer, (loc. cit.) ermittelt werden.

Für die Ermittlung dieser Aktivitäten wurden 200 µl einer 0,5 gew.-%igen Stärkelösung (analysenrein; in 50 mmol Kaliumphosphat-Puffer bei pH 7,3) mit 50 µl unterschiedlich stark verdünnter Enzymlösungen bei 25°C, 60°C, 70°C und 90°C für 3, 10 und 30 Minuten inkubiert. Nach Ablauf der Inkubationszeit wurden 250 µl eines Farbreagenz zugesetzt, welches folgende Zusammensetzung hatte:
1,0 g 3,5-Dinitro-salicylsäure
20 ml 2 mol NaOH
30 g K-Na-tartrat · 4 H₂O
Rest zu 100 ml H₂O

Der Ansatz wurde 5 Minuten bei 100°C inkubiert und anschließend mit 2,5 ml destilliertem Wasser versetzt. Die Extinktion wurde gegen einen nicht inkubierten Wert bei 546 nm bestimmt. Die Aktivität wurde als Mikromol durch enzymatische Spaltung entstandene reduzierende Enden (geeicht mit Maltose) pro Minute berechnet. Für die Berechnung wurden soweit möglich ausschließlich Ansätze herangezogen, in denen die Zunahme reduzierender Enden über den betrachteten Zeitraum linear verlaufen war. Es wurden die Aktivitäten der beschriebenen Mischung sowie einer konventionellen kommerziell verfügbaren Hochtemperaturamylase bestimmt. Die Ergebnisse sind in Tabelle 1 zusammengefaßt.

**Tabelle 1**

| Stärkeabbauende Aktivitäten einer erfindungsgemäßen Mischung und einer weiteren konventionellen HTA (Aquazym 250 L, Fa. NOVO Nordisk) bei unterschiedlichen Temperaturen im Vergleich (U = Units) | | |
|---|---|---|
| Produkt | Temperatur (°C) | Aktivität (U/ml) |
| HTA | 25 | 992 |
| Mischung | 25 | 4882 |
| HTA | 60 | 12174 |
| Mischung | 60 | 13876 |
| HTA | 70 | 17524 |
| Mischung | 70 | 13540 |
| HTA | 90 | 10480 |
| Mischung | 90 | 8151 |

Die obige Tabelle vergleicht die Aktivitäten der erfindungsgemäßen Mischung mit denen einer konventionellen HTA bei verschiedenen Temperaturen. Bei der konventionellen HTA handelt es sich nicht um die HTA, welche Bestandteil des Gemisches ist. Es handelt sich vielmehr um eine HTA, die speziell für Entschlichtungen bei erhöhten Temperaturen angeboten wird. Die Aktivitäten wurden nach der Methode von Bergmeyer (loc. cit.), bestimmt.

Sowohl das Gemisch als auch die konventionelle HTA sind bei 60°C etwa gleich aktiv. Bei 70°C ist die konventionelle HTA sogar aktiver als das Gemisch, bei 90°C gilt das gleiche.

In einem anwendungsrelevanten Versuch wurde nun überraschenderweise gefunden, daß das erfindungsgemäße Gemisch wesentlich besser zum Entschlichten geeignet ist als die konventionelle HTA. Im Gegensatz zu den Ergebnissen in der Tabelle 1 ist das Gemisch nun aktiver als die konventionelle HTA, obgleich bei hohen Temperaturen (90°C) entschlichtet wurde.

So wurden von dem erfindungsgemäßen Enzymgemisch nur 40 % der Menge zum vollständigen Entschlichten benötigt, die beim Einsatz der konventionellen HTA notwendig war. Das Gemisch arbeitet also in der Praxis weitaus effektiver als eine konventionelle HTA (Beispiele 6 und 7). Dies bedeutet in der Praxis, daß für eine erfolgreiche Entschlichtung von dem Gemisch weniger eingesetzt werden muß als von einer konventionellen HTA. Weiter halbiert die höhere Effizienz des erfindungsgemäßen Gemisches etwa die Kosten für das Entschlichtungsmittel.

### Beispiele

### Beispiel 1

Mit einer Mischung, bestehend aus 40 Vol.-% einer HTA, 10 Vol.-% einer NTA und 50 Vol.-% Wasser, wurde 100 Vol.-% Baumwolle-Gabardine mit einem Warengewicht von 270 g/m² und einer Schlichteauflage von 6 %, bestehend aus 90 % Stärke und 10 % Polyacrylat, behandelt. Entschlichtet wurde nach dem Kalt-Verweilverfahren mit 1 ml/l der erfindungsgemäßen Mischung unter Zusatz von 2 ml/l nicht-ionischem Netzhilfsmittel (90 Teile Fettalkoholpolyglykolether, 10 Teile Wasser). Die Imprägniertemperatur betrug 20°C und die Flottenaufnahme 90 %. Nach einer Verweildauer von 6 Stunden wurde in 3 Passagen 1 x 90°C alkalisch, 1 x 90°C neutral und einmal bei 30°C neutral nachgewaschen. Bewertet wurde nach der TEGEWA-Violettskala: Note 7 bis 8 (9 = vollständig entschlichtet, 1 = nicht entschlichtet).

### Beispiel 2

Im Vergleich zu Beispiel 1 wurde unter sonst gleichen Bedingungen nach dem Heiß-Verweilverfahren entschlichtet. Die Imprägniertemperatur betrug 70°C bei einer Verweildauer von 2 Stunden und einer Flottenaufnahme von 90 %. Nachwaschprozeß wie Beispiel 1. Bewertet wurde nach der TEGEWA-Violettskala: Note 8.

### Beispiel 3

Mit der erfindungsgemäßen Mischung aus Beispiel 1 wurde ein 100 % Baumwoll-Gewebe mit einem Warengewicht von 150 g/m² und einer Schlichteauflage von 9 %, bestehend aus 82 % Stärke, 13 % Polyvinylalkohol und 5 % Pillenwachs, behandelt. Entschlichtet wurde mit 2 ml der erfindungsgemäßen Mischung nach dem Heiß-Verweilverfahren unter Zusatz von 2 ml/l nicht-ionischem Netzmittel (90 Teile Fettalkoholpolyglykolether, 10 Teile Wasser). Die Ware wurde bei 60°C imprägniert und auf 90 % Flottenaufnahme abgequetscht, anschließend aufgekault und nach 3 Stunden Verweilzeit analog Beispiel 1 weiterverarbeitet. Bewertet wurde nach der TEGEWA-Violettskala: Note 9.

### Beispiel 4

Mit der erfindungsgemäßen Mischung aus Beispiel 1 wurde ein 100 % Baumwoll-Gewebe mit einem Warengewicht von 150 g/m² mit einer Schlichteauflage von 9 %, bestehend aus 82 % Stärke, 13 % Polyvinylalkohol und 5 % Pillenwachs, behandelt. Entschlichtet wurde mit 2 ml/l der erfindungsgemäßen Mischung aus Beispiel 1 nach dem Heiß-Verweilverfahren unter Zusatz von 3 ml/l nicht-ionischem Netzmittel (90 Teile Fettalkoholpolyglykolether, 10 Teile Wasser). Die Imprägniertemperatur betrug 60°C bei 90 % Flottenaufnahme und einer anschließenden Verweildauer von 12 Stunden. Das Material wurde mit Wasser bei 90°C, 60°C und zuletzt bei 30°C gewaschen. Entschlichtungsgrad nach der TEGEWA-Violettskala: Note: 9.

### Beispiel 5

Im Gegenversuch wurde die unter Beispiel 4 genannte Entschlichtung mit einer üblichen HTA mit gleicher Aktivität unter Zusatz von 3 ml/l nicht-ionischem Netzmittel durchgeführt. Der Entschlichtungsgrad war deutlich geringer (nach TEGEWA-Violettskala: Note: 5).

### Beispiel 6

Es wurde auf einer kontinuierlichen arbeitenden Vorbehandlungsanlage mit Imprägnier-, Dämpf- und Waschabteil mit 0,8 ml/l der erfindungsgemäßen Mischung aus Beispiel 1 gearbeitet. Die Ware wurde bei 70°C imprägniert und unmittelbar danach im Dämpfer bei 98°C 40 Sekunden lang behandelt. Anschließend wurde unter Zusatz von 3 g/l Soda heiß ausgewaschen und kalt gespült. Entschlichtungsgrad nach der TEGEWA-Violettskala: Note: 9.

### Beispiel 7

Ein Vergleichsversuch zu Beispiel 6 mit 0,8 ml/l einer üblichen HTA (Aquazym 250 L (Novo Nordisk), die nicht Bestandteil des erfindungsgemäßen Gemisches war, aber ähnliche Aktivität besaß, lieferte folgendes überraschende Ergebnis: Um eine Entschlichtung mit der Note 9 nach der Violettskala zu erreichen, mußte die 2,5-fache Menge (2 ml/l) der üblichen HTA eingesetzt werden, verglichen mit 0,8 ml/l gemäß Beispiel 6. Dadurch resultierte ein deutlicher Kostenvorteil beim Einsatz der erfindungsgemäßen Mischung.

**Tabelle 2**

| Aktivitätsvergleich (U = Units) | | | | | | |
|---|---|---|---|---|---|---|
| Enzym | Einsatzmenge (ml/l) | Aktivität (U)/ml | | Aktivität (U)/Einsatzmenge | | Entschlichtungsgrad nach TEGEWA-Skala |
| | | 70°C | 90°C | 70°C | 90°C | |
| Mischung | 0,8 | 13 540 | 8 151 | 10 832 | 6 521 | 9 |
| übliche HTA | 2,0 | 17 524 | 10 480 | 35 048 | 20 960 | 9 |

Die Tabelle verdeutlicht, daß die erfindungsgemäße Mischung trotz geringerer Enzymaktivität bei der meßtechnischen Feststellung ein vergleichbares anwendungstechnisches Entschlichtungsergebnis wie die übliche HTA liefert.

### Beispiel 8

Mit 1 ml/l der erfindungsgemäßen Mischung aus Beispiel 1 wurden 400 kg 100 % Baumwoll-Gewebe mit einem Warengewicht von 150 g/m² und einer Schlichteauflage von 6,7 %, bestehend aus 100 % modifizierter Stärke, auf einem Jigger entschlichtet. Das Flottenverhältnis betrug 1:6; nach 2 Passagen bei 90°C wurde in 3 Passagen heiß und in 2 Passagen kalt gespült. Entschlichtungsgrad nach der TEGEWA-Violettskala: Note: 8.

### Beispiel 9

Der Vergleichsversuch zu Beispiel 8 wurde mit 1 ml/l einer üblichen Hochtemperaturamylase durchgeführt; die so behandelte Ware zeigte nach der TEGEWA-Violettskala einen Entschlichtungsgrad mit der Note 6.

## Patentansprüche

1. Gemische verschiedener stärkeabbauender Enzyme (Amylasen), die aus mindestens einer Hochtemperaturamylase (HTA) und mindestens einer Niedertemperaturamylase (NTA) im Aktivitätsverhältnis von HTA zu NTA von 10 % : 90 % bis 90 % : 10 % bestehen und mindestens 60 % ihrer maximalen Enzymaktivität im Temperaturbereich von 30 bis 90°C besitzen und die weiterhin mit Wasser verdünnt und mit üblichen Zusatzmitteln versetzt werden können.

2. Gemische nach Anspruch 1 mit einem Aktivitätsverhältnis von HTA zu NTA von 20 % : 80 % bis 80 % : 20 %, bevorzugt 30 % : 70 % bis 70 % : 30 %, besonders bevorzugt 40 % : 60 % bis 60 % : 40 %.

3. Gemische nach Anspruch 1 mit mindestens 80 % der Summe ihrer maximalen Aktivität im Temperaturbereich von 45 bis 75°C.

4. Verfahren zum Entschlichten von mit Stärke geschlichteten Textilien durch Behandlung der Textilien mit stärkeabbauenden Enzymen (Amylasen) und anschließendem Spülen, dadurch gekennzeichnet, daß man die Behandlung bei 30 bis 98°C und einem Gemisch gemäß Anspruch 1 vornimmt.
